# FASCICULE DE BREVET EUROPEEN

(11) **EP 1 150 736 B1**
(45) Date de publication et mention de la délivrance du brevet: **23.06.2004**
(21) Numéro de dépôt: 00976108.1
(22) Date de dépôt: 06.11.2000
(51) Int. Cl.: A61M 25/01

(54) **DISPOSITIF DE COMMANDE MANUELLE D'UN GUIDE CHIRURGICAL**
MANUELLE STEUERVORRICHTUNG FÜR EINEN CHIRURGISCHEN FÜHRUNGSDRAHT
MANUAL CONTROL DEVICE FOR A SURGICAL GUIDE

(30) Priorité: 10.12.1999 FR 9915629
(43) Date de publication de la demande: 07.11.2001
(73) Titulaire: SEDAT, 69540 Irigny (Rhône) (FR)
(72) Inventeur: DELAY, Jean-Pascal, F-69130 Ecully (FR)
(74) Mandataire: Blot, Philippe Robert Emile
(86) Numéro de dépôt international: PCT/FR2000/003083
(87) Numéro de publication internationale: WO 2001/041860

(56) Documents cités:
- WO-A-97/11736
- WO-A-97/18850
- US-A- 4 829 999
- US-A- 4 973 329
- US-A- 5 325 868

## Description

La présente invention concerne un dispositif de commande manuelle d'un guide chirurgical, du type comportant un corps muni d'un conduit de passage du guide chirurgical, et un organe de blocage axial du guide chirurgical par rapport au corps, lequel organe est déplaçable entre une position de blocage du guide chirurgical et une position de libération du guide chirurgical. Voir par exemple dans WO-A-9711736.

Un tel dispositif de commande manuelle est utilisé pour faciliter le maniement d'un guide mis en place lors d'interventions en angioplastie et en angiographie. Un tel guide est constitué d'un fil souple de grande longueur, celle-ci pouvant atteindre deux mètres. Ce guide a un diamètre très petit, compris généralement entre 0,01 pouce et 0,045 pouce (0,25 mm à 1,15 mm).

Le dispositif de commande manuelle monté sur le guide permet, lors d'une intervention, de pousser le guide, de le tirer, et de le tourner sur lui-même, après que l'extrémité de celui-ci a été introduite dans le corps humain et notamment à l'intérieur d'un conduit vasculaire.

Le déplacement du guide est facilité puisque le dispositif de commande manuelle constitue un organe solidaire du guide et dont les dimensions facilitent la prise en main par rapport à une action directe sur le guide qui est trop fin pour être saisi facilement.

Des dispositifs de commande manuelle de guide sont déjà connus et couramment utilisés. Ceux-ci comportent un corps traversé par un conduit de passage du guide. Un organe de blocage est engagé autour du guide. Il est adapté pour être vissé sur le corps en comprimant radialement le guide, assurant ainsi la solidarisation du guide par rapport au corps.

Un tel dispositif est relativement complexe et d'un coût de fabrication élevé puisqu'il comporte plusieurs pièces et que ces pièces doivent être ajustées avec précision, afin de permettre un vissage efficace.

Il est par ailleurs connu des dispositifs de commande manuelle comportant un corps sur lequel coulisse un organe de blocage qui assure l'immobilisation du guide chirurgical par effet de coin. Pour le blocage du guide, l'organe de blocage est sollicité par une rampe formant came ménagée sur le corps.

Ce dispositif est également complexe à réaliser, puisqu'il nécessite la mise en oeuvre de plusieurs éléments et de moyens de guidage précis des éléments les uns par rapport aux autres, afin de fonctionner de matière satisfaisante.

Par ailleurs, ces dispositifs sont d'un maniement relativement délicat pour le chirurgien, puisque la libération du guide chirurgical et son immobilisation n'est pas aisée.

L'invention a pour but de fournir un dispositif de commande manuelle pour guide chirurgical ayant un faible coût de fabrication et permettant un usage simple.

A cet effet, l'invention a pour objet un dispositif de commande manuelle d'un guide chirurgical, du type précité, caractérisé en ce que ledit organe de blocage est venu de matière avec le corps.

Suivant des modes particuliers de réalisation, le dispositif de commande manuelle comporte l'une ou plusieurs des caractéristiques suivantes :
- ledit organe de blocage comporte une plage d'appui transversal sur le guide chirurgical et des moyens de sollicitation de ladite surface d'appui transversalement à l'axe du conduit ménagé dans le corps ;
- lesdits moyens de sollicitation comportent des moyens de rappel élastique de l'organe de blocage vers sa position de blocage ;
- l'organe de blocage comporte un conduit de réception du guide, la plage d'appui transversal sur le guide étant définie sur la surface dudit conduit ;
- ledit corps comporte un logement de réception de l'organe de blocage en position de blocage, lequel logement forme une interruption du conduit, lequel conduit délimite, de part et d'autre du logement, deux plages d'appui transversal du guide chirurgical ;
- il comporte un bras portant ledit organe de blocage, lequel bras est articulé par rapport au corps ; et
- le bras portant l'organe de blocage est relié au corps par une région de liaison déformable élastiquement constituant lesdits moyens de rappel élastique de l'organe de blocage vers sa position de blocage.

L'invention sera mieux comprise à la lecture de la description qui va suivre, donnée uniquement à titre d'exemple et faite en se référant aux dessins sur lesquels :

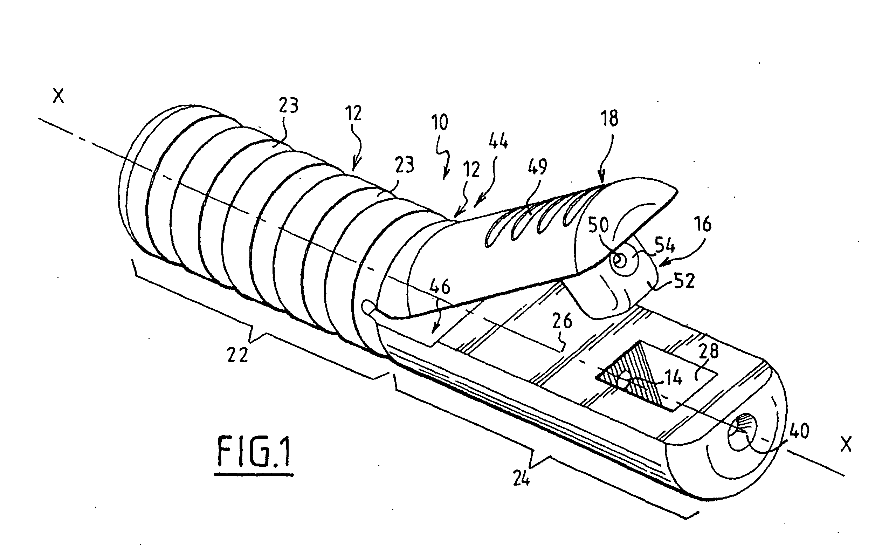

- la figure 1 est une vue en perspective de trois-quarts d'un dispositif de commande manuelle selon l'invention représenté seul ;
- la figure 2 est une vue en coupe longitudinale du dispositif de la figure 1 au repos, en l'absence de guide chirurgical ;
- la figure 3 est une vue en coupe longitudinale du dispositif de commande manuelle dans sa position d'introduction du guide chirurgical ou de libération de celui-ci ; et
- la figure 4 est une vue en coupe longitudinale du dispositif de commande manuelle selon l'invention en position de blocage d'un guide chirurgical.

Le dispositif 10 selon l'invention, représenté aux figures, est destiné à la commande manuelle d'un guide chirurgical. Il présente une forme générale allongée sensiblement cylindrique d'axe X-X. Il comporte, d'une part, un corps 12 traversé de part en part axialement par un conduit 14 et, d'autre part, un organe 16 de blocage du guide chirurgical par rapport au corps. Cet organe 16 est venu de matière avec un bras de commande 18, lui-même venu de matière avec le corps 12.

Plus précisément, le corps 12 comporte un tronçon de préhension 22 à la surface extérieure duquel sont ménagées des gorges 23 facilitant le maniement du dispositif.

Le tronçon de préhension 22 est prolongé par un tronçon 24 de blocage du guidage chirurgical. Ce tronçon de forme extérieure essentiellement cylindrique présente sur toute sa longueur un méplat 26 en regard du bras 18.

Le tronçon 24 comporte un logement 28 de réception de l'organe de blocage 16. Le logement 28 débouche au travers du méplat 26, et traverse transversalement le tronçon de blocage 24. Il est délimité par quatre surfaces planes perpendiculaires les unes aux autres. Le logement 28 est ménagé au voisinage de l'extrémité libre du tronçon 24.

Le conduit 14 s'étend suivant toute la longueur du corps. Il comporte un tronçon principal 32 s'étendant suivant toute la longueur du tronçon de préhension 22. Il se prolonge dans le tronçon de blocage 24 et débouche perpendiculairement suivant une face latérale du logement 28. Ce tronçon principal est complété par un tronçon secondaire 34 ménagé entre l'extrémité libre du tronçon de blocage 24 et le logement 28. Le conduit 14 présente, de part et d'autre du logement 28, des tronçons 36, 38 de section réduite. Ces tronçons ont un diamètre très légèrement supérieur au diamètre du guide destiné à y être maintenu.

En outre, à ses extrémités débouchantes aux extrémités du corps 12, le conduit 14 présente des tronçons de section progressivement croissante vers l'extérieur formant des cônes de centrage 40, 42 facilitant l'insertion du guide chirurgical.

Comme illustré aux figures, le bras 18 et l'organe de blocage 16 sont tous deux venus de matière avec le corps 12. A cet effet, le dispositif de commande manuelle est réalisé par injection de matière plastique, en une seule fois, dans un moule de forme adapté.

Le bras 18 est relié au corps 12 par une région de liaison 44 formant charnière qui est déformable élastiquement. Afin de faciliter la déformation de la région 44, un dégagement 46 est prévu dans le méplat 26 en regard de la région de liaison 44.

Le bras 18 présente une longueur sensiblement égale à celle du tronçon de blocage 24. Il présente une surface extérieure généralement cylindrique et une surface plane 48 disposée en regard du méplat 26. A sa surface extérieure, il présente des profils en creux 49 facilitant l'action manuelle sur le bras.

Au repos, c'est-à-dire en l'absence de toute contrainte, le bras 18 est écarté du corps et définit avec le méplat 26 un angle sensiblement égal à 30°. Dans cette position, l'organe de blocage 16 est en dehors du logement 28. Cette position du bras correspond à la configuration dans laquelle est moulé le dispositif de commande.

L'organe de blocage 16 fait saillie par rapport à la surface plane 48 du bras. Il est disposé au voisinage de l'extrémité libre du bras, en regard du logement 28. Il a des dimensions adaptées pour qu'il puisse être reçu dans ce logement.

L'organe de blocage 16 présente une forme sensiblement parallélépipédique, ses arêtes transversales étant arrondies.

De plus, l'organe 16 est traversé de part en part suivant l'axe du bras par un conduit 50 débouchant suivant des faces transversales 52. Ce conduit 50 présente un diamètre sensiblement égal à celui des tronçons de section réduite 36 et 38 du conduit traversant le corps. A son extrémité orientée du côté de l'extrémité libre du bras, le conduit 50 est muni d'un cône de centrage 54 facilitant l'introduction du guide chirurgical.

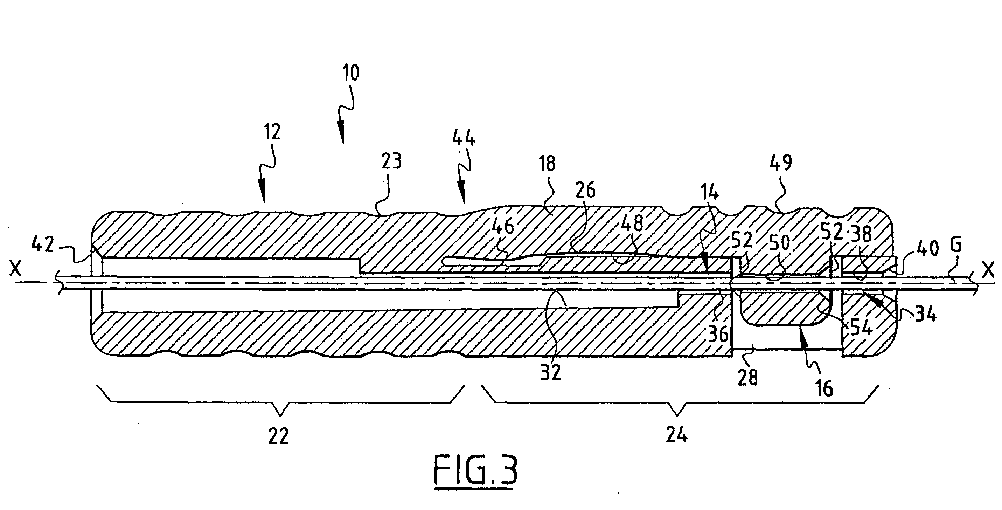

Le conduit 50 est ménagé dans une position telle que, lorsque le bras 18 est sollicité vers le corps et que la région de liaison 44 est déformée de sorte que le bras 18 soit en appui sur le méplat 26, le conduit 50 et les tronçons 36 et 38 du conduit 14 soient sensiblement coaxiaux, comme illustré sur la figure 3.

Lorsque le bras 18 est en appui sur le méplat 26, l'organe de blocage 18 se trouve dans une position d'introduction du guide chirurgical noté G, ou dans une position de libération du guide permettant le déplacement axial, et à rotation, du dispositif de commande par rapport au guide chirurgical.

Le dispositif de commande manuelle représenté aux figures s'utilise de la manière suivante.

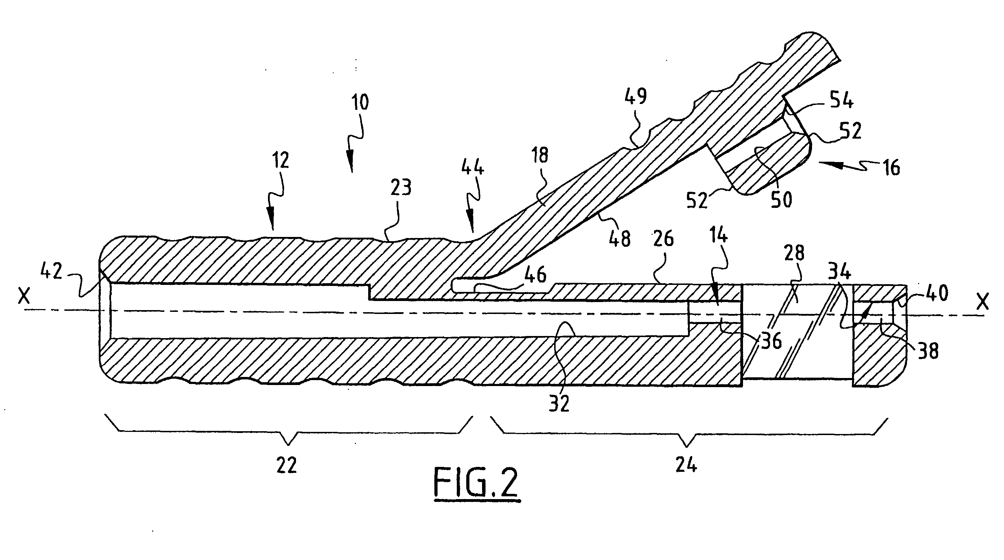

Initialement, le dispositif est dans sa configuration de repos illustrée sur la figure 2.

Afin de permettre l'introduction du guide chirurgical, l'organe 16 de blocage 16 est amené dans sa position de libération du guide chirurgical, telle qu'illustrée sur la figure 3, dans laquelle le conduit 50 est coaxial aux tronçons 36 et 38.

A cet effet, la région 44 de liaison du bras est alors déformée élastiquement, par pression manuelle sur le bras 18 jusqu'à ce que celui-ci s'applique contre le tronçon de blocage du corps.

Le guide chirurgical peut alors être introduit au travers du conduit 14. Cette introduction s'effectue avantageusement par l'ouverture ménagée à l'extrémité libre du tronçon de blocage 24.

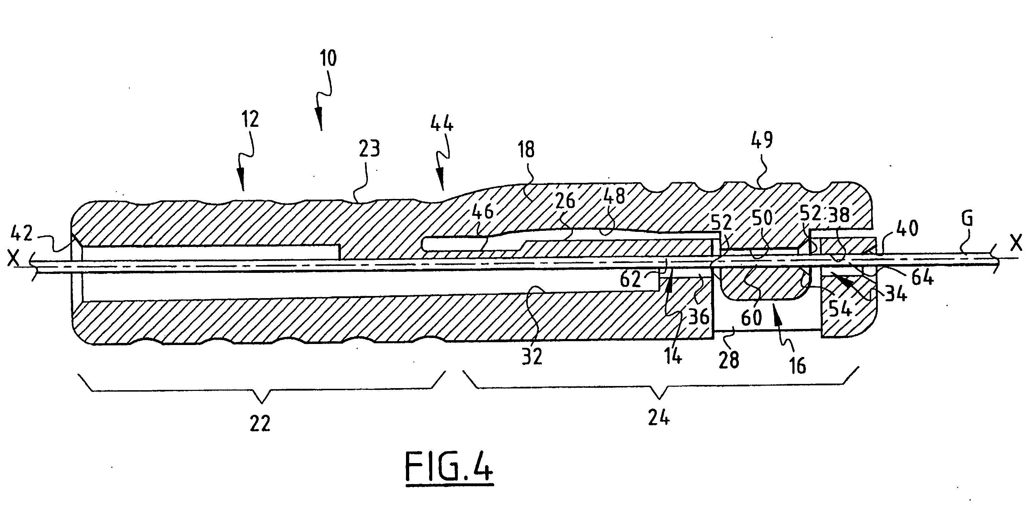

Après mise en place du dispositif de commande manuelle autour du guide chirurgical dans la région souhaitée, la pression exercée sur le bras 18 est interrompue. Ainsi, sous l'action de relâchement de la région de liaison 44 préalablement déformée, le bras 18 est rappelé élastiquement vers sa position de repos. L'organe de blocage 16 sollicite alors transversalement le guide chirurgical G. Ainsi, une plage, notée 60 sur la figure 4, de la surface latérale du conduit 50 sollicite transversalement le guide chirurgical G. Cette place 60 est formée par la région du conduit 50 éloignée du bras 18 et est tournée vers celui-ci.

Au contraire, des plages des tronçons 36 et 38, notées 62 et 64, disposées du côté du bras 18 constituent des surfaces d'appui du guide chirurgical G. Ainsi, sous l'action de la force transversale de rappel élastique, le guide chirurgical G se trouve bloqué, d'une part, entre les plages 60 et 62 et, d'autre part, entre les plages 60 et 64. Du fait de ce blocage, le guide chirurgical est immobilisé à la fois axialement et en rotation par rapport au dispositif de commande.

Dans cette position de blocage occupée par l'organe 16, le conduit 50 et les tronçons 36 et 38 sont désalignés.

Le guide chirurgical G peut ainsi être facilement manipulé par le chirurgien qui maintient le dispositif de commande entre ses doigts, sans appuyer sur le bras 18.

Lorsque le chirurgien souhaite faire coulisser le dispositif de commande manuelle le long du guide, ou déplacer angulairement le dispositif de commande par rapport au guide, il exerce manuellement une force d'appui sur le bras 18 de manière à amener l'organe de blocage 16 dans sa position de libération illustrée sur la figure 3. L'alignement du conduit 50 et des tronçons 36 et 38 met un terme au coincement du guide G qui est alors libre de se déplacer.

La présence, de part et d'autre de l'organe de blocage 16, des plages d'appui 62 et 64 assure une répartition des forces de sectionnement du guide chirurgical en deux régions suivant la longueur de celui-ci, ces régions étant ménagées de part et d'autre de l'organe 16. Ainsi, les dégradations mécaniques apportées au guide sont réduites.

De plus, la présence des deux plages d'appui 62, 64 évite de créer des déformations locales du guide chirurgical, celui-ci restant essentiellement rectiligne dans sa région de coincement.

Toutefois, un tel coincement pourrait également être obtenu même en supprimant l'un des tronçons 36 et 38, le guide G n'étant alors plus bloqué qu'entre un couple de plages opposées 60, 62 ou 60, 64.

De même, le conduit 50 peut être remplacé par tout profil permettant le passage du guide G et délimitant au moins une plage d'appui transversal sur le guide, cette surface pouvant être plane par exemple.

On comprend que l'utilisation du dispositif de commande manuelle décrit ici, qui est réalisé d'une seule pièce puisque l'organe de blocage 16 est venu de matière avec le corps, réduit le coût de fabrication du dispositif et augmente la fiabilité de celui-ci. De plus, la manipulation du dispositif est extrêmement simple, le guide chirurgical étant automatiquement bloqué par rapport au corps par le rappel élastique exercé sur l'organe de blocage 16 par la région déformable assurant la liaison du bras 18.

La libération du guide chirurgical s'effectue également de manière très simple puisqu'il suffit d'exercer une pression manuelle sur le bras 18 et ce transversalement au guide chirurgical. Un tel appui sur le bras 18 s'effectue de manière naturelle pour le chirurgien, contrairement au vissage d'un organe dé blocage ou au déplacement axial de celui-ci par rapport au corps qui nécessitent une agilité importante.

Avec le dispositif décrit ici, la pression exercée par l'organe de blocage sur le guide chirurgical est fixée par la déformation élastique initiale du bras 18. Cette pression est donc constante et ne dépend pas de l'effort appliqué par le chirurgien contrairement aux dispositifs de l'état de la technique où l'effort de vissage ou de coincement par déplacement axial d'un bouton dépendent de l'action du chirurgien. Ainsi, tout risque de mauvais blocage par un effort de coincement insuffisant ou de détérioration du guide par un effort de coincement excessif est évité, l'effort de coincement étant défini par la force constante de rappel élastique du bras.

## Revendications

1. Dispositif (10) de commande manuelle d'un guide chirurgical (G), comportant un corps (12) muni d'un conduit (14) de passage du guide chirurgical (G), et un organe (16) de blocage axial du guide chirurgical par rapport au corps, lequel organe (16) comporte un conduit (50) de réception du guide chirurgical et **caractérisé en ce que** ledit organe (16) est déplaçable entre une position de blocage du guide chirurgical dans laquelle les conduits (14, 50) ménagés dans le corps (12) et dans l'organe de blocage (16) sont désalignés et une position de libération du guide chirurgical dans laquelle les conduits (14, 50) ménagés dans le corps (12) et dans l'organe de blocage (16) sont alignés, le dispositif comportant des moyens (44) de rappel élastique de l'organe de blocage (16) vers sa position de blocage, ledit organe de blocage (16) est venu de matière avec le corps (12).

2. Dispositif de commande manuel selon la revendication 1, **caractérisé en ce que** ledit corps (12) comporte un logement (28) de réception de l'organe de blocage (16) en position de blocage, lequel logement (28) forme une interruption du conduit (14), lequel conduit (14) délimite, de part et d'autre du logement (28), deux plages (62, 64) d'appui transversal du guide chirurgical (G).

3. Dispositif de commande selon la revendication 2, **caractérisé en ce que**, au repos, l'organe de blocage (16) est en-dehors du logement (28) de réception de l'organe de blocage.

4. Dispositif de commande manuelle selon l'une quelconque des revendications précédentes, **caractérisé en ce qu'**il comporte un bras (18) portant ledit organe de blocage (16), lequel bras (18) est articulé par rapport au corps (12).

5. Dispositif de commande manuelle selon la revendication 4, **caractérisé en ce que** le bras (18) portant l'organe de blocage est relié au corps (12) par une région de liaison (44) déformable élastiquement constituant lesdits moyens de rappel élastique de l'organe de blocage (16) vers sa position de blocage.

## Patentansprüche

1. Manuelle Steuervorrichtung (10) für einen chirurgischen Führungsdraht (G), mit einem einen Durchlasskanal (14) für einen den chirurgischen Führungsdraht (G) aufweisenden Körper (12), und mit einer Klemmeinrichtung (16) zum axialen Festklemmen des chirurgischen Führungsdrahts (G) in Bezug auf den Körper, wobei diese Einrichtung (16) einen Aufnahmekanal (50) für den chirurgischen Führungsdraht (G) aufweist, und **dadurch gekennzeichnet ist, dass** diese Einrichtung (16) zwischen einer Klemmstellung des chirurgischen Führungsdrahts (G), in welcher die in dem Körper (12) und in der Klemmeinrichtung (16) angeordneten Kanäle (14, 50) nicht fluchten, und einer gelösten Stellung für den chirurgischen Führungsdraht (G), in welcher die in dem Körper (12) und in der Klemmeinrichtung (16) angeordneten Kanäle (14, 50) fluchten, verstellbar ausgebildet ist, wobei die Vorrichtung Einrichtungen (44) zum elastischen Rückstellen der Klemmeinrichtung (16) in Richtung auf ihre Klemmstellung aufweist, wobei die Klemmeinrichtung (16) zusammen mit dem Körper (12) einstückig ausgebildet ist.

2. Manuelle Steuervorrichtung nach Anspruch 1, **dadurch gekennzeichnet, dass** der Körper (12) einen Aufnahmeraum (28) für die Klemmeinrichtung (16) in Klemmstellung aufweist, dieser Aufnahmeraum (28) eine Unterbrechung des Kanals (14) bildet, und dieser Kanal (14) beiderseits des Aufnahmeraums (28) zwei hindurchlaufende Auflagebereiche (62, 64) für den chirurgischen Führungsdraht (G) begrenzt.

3. Manuelle Steuervorrichtung nach Anspruch 2, **dadurch gekennzeichnet, dass** sich die Klemmeinrichtung (16) in unbenutzter Stellung außerhalb des Aufnahmeraums (28) befindet.

4. Manuelle Steuervorrichtung nach einem der vorhergehenden Ansprüche, **dadurch gekennzeichnet, dass** sie einen Arm (18) aufweist, welcher die Klemmeinrichtung (16) trägt, wobei dieser Arm (18) in Bezug auf den Körper (12) gelenkig beweglich ausgebildet ist.

5. Manuelle Steuervorrichtung nach Anspruch 5, **dadurch gekennzeichnet, dass** der Arm (18), der die Klemmeinrichtung (16) trägt, mit dem Körper (12) über einen Verbindungsbereich (44) verbunden ist, welcher elastisch verformbar in Richtung auf seine Klemmstellung ausgebildet ist, wobei dieser die Einrichtungen zum elastischen Rückstellen der Klemmeinrichtung (16) bildet.

## Claims

1. Manual control device (10) for a surgical guide (G), comprising a body (12) provided with a duct (14) for the surgical guide (G) to pass through, and an axial blocking member (16) for axially blocking the surgical guide relative to the body, said member (16) comprising a duct (50) for accommodating the surgical guide, **characterised in that** said member (16) is displaceable between a blocking position for the surgical guide wherein the ducts (14, 50) provided in the body (12) and in the blocking member (16) are out of alignment and a release position for the surgical guide wherein the ducts (14, 50) provided in the body (12) and in the blocking member (16) are aligned, the device comprising resilient return means (44) for returning the blocking member (16) to its blocking position, said blocking member (16) being integrally formed with the body (12).

2. Manual control device (10) according to claim 1, **characterised in that** the body (12) comprises a recess (28) for receiving the blocking member (16) in the blocking position, said recess (28) forming an interruption to the duct (14), said duct (14) defining, on either side of the recess (28), two transverse support surfaces (62, 64) for the surgical guide (G).

3. Control device according to claim 1, **characterised in that** in the resting position the blocking member (16) is outside the recess (28) for receiving the blocking member.

4. Manual control device according to one of the preceding claims, **characterised in that** it comprises an arm (18) carrying said blocking member (16), said arm (18) being articulated relative to the body (12).

5. Manual control device according to claim 4, **characterised in that** the arm (18) carrying the blocking member is connected to the body (12) by an elastically deformable connecting region (44) which constitutes the resilient means for returning the blocking member (16) to its blocking position.
